(19) Europäisches Patentamt
European Patent Office
Office européen des brevets

(11) Publication number: **0 085 283**
**A1**

(12) **EUROPEAN PATENT APPLICATION**

(21) Application number: 82830298.4

(22) Date of filing: 13.12.82

(51) Int. Cl.³: C 07 C 103/52
A 61 K 37/02

(30) Priority: 15.12.81 IT 4991281

(43) Date of publication of application:
10.08.83 Bulletin 83/32

(84) Designated Contracting States:
AT BE CH DE FR GB LI LU NL SE

(71) Applicant: Società Italo-Britannica L. Manetti - H. Roberts & C.
Via A. da Noli 4
I-50127 Firenze(IT)

(72) Inventor: Orzalesi, Giovanni
Via di Camerata 71
I-50133 Firenze(IT)

(72) Inventor: Volpato, Ivo
Via di Barbano 14
I-50129 Firenze(IT)

(72) Inventor: De Regis, Massimo
Viale Giulio Cesare 11
I-50019 Sesto Fiorentino(IT)

(74) Representative: Bazzichelli, Alfredo et al,
c/o Società Italiana Brevetti Piazza Poli 42
I-00187 Roma(IT)

(54) **Pyroglutamyl-tryptophan derivatives, process for their preparation and their therapeutic applications.**

(57) Pyroglutamyltrypotophan with formula

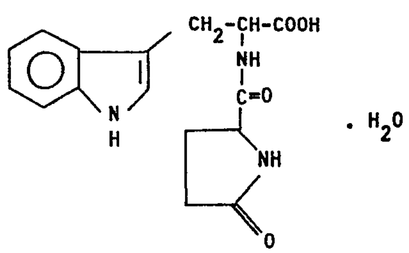

obtained as the monohydrate crystal and the pyroglutamyl-tryptophan ethyl ester are pharmaceutically active agents with antipsychotic and neurotropic action.

EP 0 085 283 A1

0085283

SOCIETA' ITALO-BRITANNICA L. MANETTI - H. ROBERTS & C.

# Pyroglutamyl-tryptophan derivatives, process for their preparation and their therapeutic applications

## Specification

The present invention refers to a new organic molecule identified as pyroglutamyltryptophan, with formula:

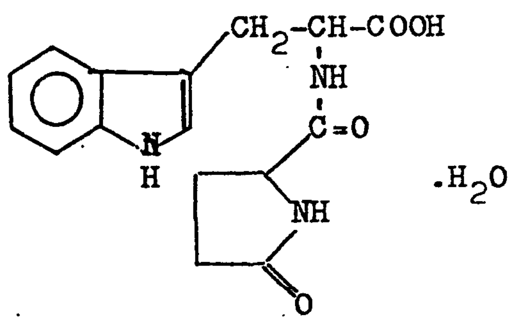

where said molecule is pharmaceutically active as a neurotropic and antipsychotic agent.

The present invention also refers to a process for the synthesis of this new molecule in crystalline form: more particularly, it concerns the compound prepared by peptide condensation of levorotatory, dextrorotatory or racemic tryptophan with pyroglutamic acid (also levorotatory, dextrorotatory ro racemic), as well as a process for the preparation of this molecule and the useful pharmaceutical compositions containing it.

The present invention also refers in particular to the

ethyl ester derivative of pyroglutamyltryptophan, obtained as an intermediate product in the process mentioned above, as well as the pharmacological properties of said ethyl ester, which are the same as those of the molecule with the free carboxyl group.

Taken singly, the two amino acids which form the dipeptide molecule are already known.

Tryptophan is an essential amino acid, the metabolism of which leads mainly to the synthesis of nicotinic acid, which in turn becomes part of the pyridinic nucleotides NAD and NADP, coenzymes in a large number of oxidation-reductions.

Secondly, tryptophan is a precursor of the neurotransmitter 5-hydroxytryptophan (serotonin). The serotoninergic neurons are correlated with many nervous functions, including mood (a), sleep (b) and the pain function (c).

(a) The hypothesis that a deficinecy of serotoninergic neurotransmitters in the central nervous system can cause depression is based on the consideration that antidepressant drugs activate these neurotransmitters while antiserotoninergic compounds induce depression.

(b) Serotoninergic neurons are known to be involved in the physiology and biochemistry of sleep: among other

0085283

| Number of C atoms | integral | Hz | ppm | |
|---|---|---|---|---|
| 1 | 28.75 | 3547.4 | 177.36 | |
| 2 | 36.72 | 3461.4 | 173.06 | |
| 3 | 36.07 | 3449.0 | 172.44 | |
| 4 | 22.42 | 2720.5 | 136.02 | |
| 5 | 18.28 | 2541.7 | 127.08 | |
| 6 | 69.65 | 2472.2 | 123.60 | |
| 7 | 72.70 | 2417.0 | 120.84 | |
| 8 | 79.96 | 2366.2 | 118.30 | |
| 9 | 74.07 | 2361.1 | 118.05 | |
| 10 | 69.71 | 2225.8 | 111.28 | |
| 11 | 36.71 | 2192.4 | 109.61 | |
| 12 | 66.75 | 1104.8 | 55.24 | |
| 13 | 61.21 | 1057.0 | 52.84 | |
| 22 | 71.37 | 578.6 | 28.93 | |
| 23 | 50.10 | 537.7 | 26.88 | |
| 24 | 68.69 | 501.6 | 25.07 | |
| 25 | 21.20 | 0.0 | 0.00 | TMS |

the C atoms being numbered as follows:

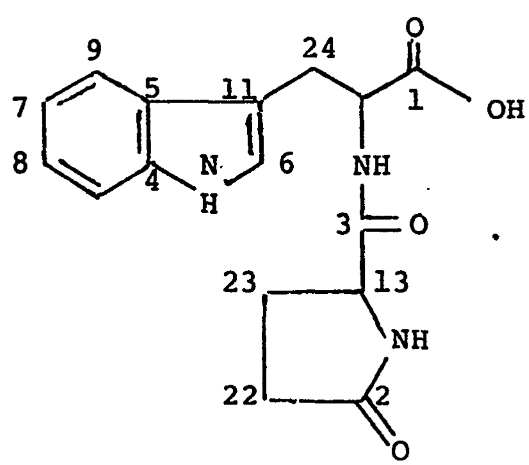

4. Pyroglutamyl-tryptophan ethyl ester obtained as an intermediate product by the process claimed in claim 1 or 2.

5. Pyroglutamyl-tryptophan monohydrate as claimed in claim 3, for use as a neurotropic agent.

6. Pyroglutamyl-tryptophan monohydrate as claimed in claim 3, for use as an antipsychotic agent.

7. Pyroglutamyl-tryptophan ethyl ester for use as a neurotropic agent.

8. Pyroglutamyl-tryptophan ethyl ester for use as an antipsychotic agent.

9. Pharmaceutical composition containing a pharmaceutically effective amount of pyroglutamyl-tryptophan monohydrate as claimed in claim 3, and pharmaceutically compatible excipients.

10. Pharmaceutical composition containing a pharmaceutically effective amount of pyroglutamyl-tryptophan ethyl ester, and pharmaceutically compatible excipients.

Nicotinic acid deficiency gives rise to a known symptomatology which in addition to dermatitis and disturbances of the gastrointestinal system, is characterized by signs of cerebral compromise, such as irritability, insomnia, depression, poor memory, headaches.

Pyroglutamic acid is also a natural amino acid, with the following interesting properties.

(1) It is found as the N-terminal residue at the beginning of the chain of various neuro-hormones (like TRH and LS-RH).

Its presence may be interpreted both as a defense against hexopeptidase attack and as a means to facilitate passage through the membrane, since it is more lipophilic than other chemically related amino acids like glutamic acid.

(2) It is involved in the $\gamma$-glutamylic cycle, proposed amont other things as the general mecahnism for amino acid transport in the cells. This cycle includes the synthesis and breakdown of glutathione, and the enzymes involved in it are present in various tissues in the mammary gland, encephalon and skin.

An active intermediate in the cycle, pyroglutamate also enhances the rate of glutathione synthesis and turnover.

Consistent with these observations, it has been shown that in the presence of pyroglutamate, amino acid absorption by the cells is increased. In addition to being implicated in the mechanism of amino acid transport, glutathione has an important role in the protection of the cellular membranes and proteins, maintaining the sulfidrylic groups and interacting with hydrogen peroxides and free radicals.

It has also been observed that glutathione can give rise to a small accumulation of triglycerides in hepatic steatosis.

Thus, as an active intermediate in the cycle involving the synthesis and breakdown of glutathione, pryoglutamate plays a role as detoxifier and liver protector. It has recently been reported that chronic treatment with ethanol leads to inhibition of tryptophan-pyrrolase (limiting enzyme in the process leading to formation of nicotinic acid) and that this inhibition is reversed by pyroglutamic acid. This suggests its eventual application in conditions of alcohol intoxication.

(3) It has been known for some time that glutamic acid can be formed in the encephalon starting from proline, in a process with pyroglutamic acid (5-oxo-proline) as an intermediate. On the other hand, it is known that one of the roles of glutamic acid in the central nervous system (in addition to being a GABA precursor and a regulator of ammonia levels) is that of neurotransmitter exciter; it could therefore possibly be used in cases of cerebral depression. However, glutamic acid passes the hematoencephalic barrier only with great difficulty, while pyroglutamic acid, given its cyclization, is more lipophilic and so passes the membrane more easily, to the point of being used as carrier or vehicle for some hormones. Thus administration of pyroglutamic acid may lead to increased encephalic levels of glutamic acid; this may be an important mecahnism by which pyroglutamate exerts its trophic action in the central nervous system.

Therefore, administration of pyroglutamic acid brings it to increased levels, and this increase may be helpful in cases of psychic fatigue, dementia and intellectual deficit.

The quite surprising discovery has now been made that the bond between pyroglutamic acid and tryptophan facilitates tryptophan passage through the hematoencephalic barrier and so access to nervous system cells. It has been shown that pyroglutamyltryptophan easily reaches the brain cells in concentrations sufficient to re-establish the compromised physiological equilibrium, giving a charge of the two amino acids greater than does administration of the individual components. This leads

to increased 5-hydroxytryptophan levels due to the charge of tryptophan in cases where this increase is considered useful, for example: depressive conditions, sleep disturbances, hemicrania and central nervous system origin headaches.

The dipeptide molecule pyroglutamyl tryptophan would at other : levels be useful in cases of hepatic pathologies, alcoholism, hyperamnoiemia.

## Preparation

It has been found that by condensing pyroglutamic acid (I) as such or as its N-carbobenzoxy derivative with tryptophan (II)

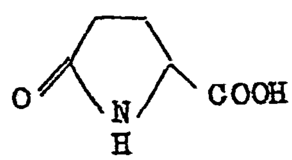

(I)

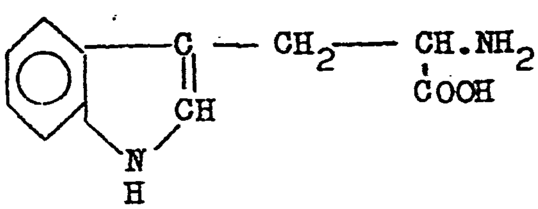

(II)

in the presence of dicyclohexylcarbodiimide, a dipeptide molecule is obtained corresponding to the followin pyroglutamyltryptophan (III):

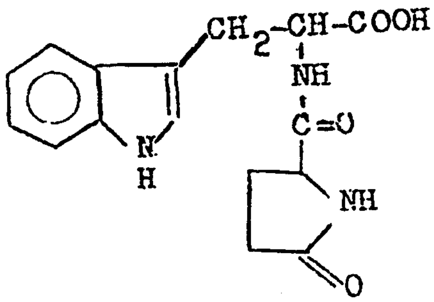

(III)

As a variant, the same molecule may be obtained with the mixed anhydride method, as shown by the example in the present invention.

During the synthesis procedure, the ethyl ester of pyroglutamyltryptophan is also obtained, corresponding to formula (IV):

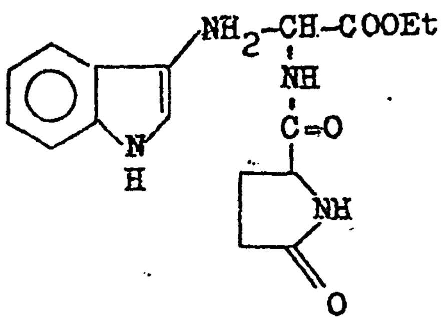

(IV)

This ester is also active in the pharmacological reactions mentioned above with reference to the monohydrate acid molecule.

Thus the following form the object of the present invention: pyroglutamyltryptophan, molecular formula $C_{16}H_{17}N_3O_4 \cdot H_2O$, molecular weight 333g/mol; pyroglutamyltryptophan ethyl ester, $C_{18}H_{21}N_3O_4$, molecular weight 343g/mol, based on the structures reported above.

For the sake of brevity in the following description the

new compounds defined above will be indicated with the generic names "pGlu-Trp" and "pGlu-Trp-ethyl ester".

The chemical structure of the molecules which form the object of the present invention may be determined on the basis of the following considerations, taking into account the fact that they are purely indicative and non-limiting to the present invention, since obviously another crystalline form than the monohydrate of "pGlu-Trp" and a different alkyl substitution than ethyl for the ester derivative, would lead to compounds with the same activity characteristics as those claimed in the present invention and should not be considered new with respect to it.

The "pGlu-Trp" monohydrate according to the present invention is a white oderless crystalline powder, air stable and readily oxidized by water, with a 130-132$^{o}$C Kofler melting point.

The structure was determined by means of elementary analysis (C;H,N), IR and NMR ($^{13}$C) spectra.

Elementary analysis

| | %N | %C | %H |
|---|---|---|---|
| Theoretical | 12.60 | 57.65 | 5.74 |
| Found | 12.45 | 57.59 | 5.66 |

## IR spectrum

The following characteristic bands were clearly visible:

| | |
|---|---|
| 3600 $cm^{-1}$ | $H_2O$ of crystallization |
| 3400 $cm^{-1}$ | indolic N-H |
| 3290-3310 $cm^{-1}$ | lactam and amide N-H |
| 1730 $cm^{-1}$ | free carboxyl group |
| 1630-1660 $cm^{-1}$ | amide and lactam carbonyl |

## $^{13}C$ NMR spectrum

The $^{13}C-\{H\}$ (noise decoupling) spectrum was obtained using Fourier transform techniques using a Varian Fourier Transform 80-A spectrometer operating at a working frequency of 17,750,800 Hz, using an impulse of 11 sec (corresponding to a 90° flip angle). The internal standard for the frequency of the field was provided by using deuterated solvents.

The sample was dissolved in 2.5ml of DMSO-$d_6$ in a 10mm NMR tube and inserted in a $\omega$-probe with a 10mm insert at 25°C.

All $^{13}C$ chemical shifts are reported with respect to the $^{13}C$ resonance frequency of TMS, used as internal standard. Signal assignment for the various carbon atoms in the molecule is in perfect agreement with the shift interval reported in the correlation tables for

$^{13}C$-NMR signals; on the other hand, a study of compounds representing the various fragments of the molecule reveals a striking agreement between the chemical shift of the $^{13}C$-NMR signals of the reference structures and those of the test sample. The carbon atoms were numbered as shown in the following formula:

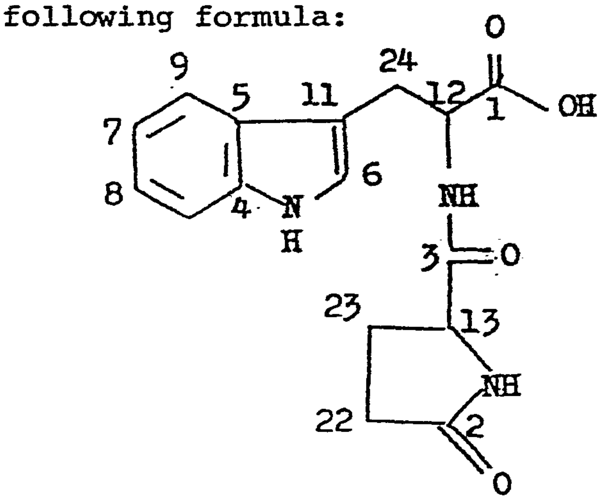

The corresponding resonance values are reported in the following table:

Table 1

| Carbon atom No. | Integral | (Hz) | (ppm) | |
|---|---|---|---|---|
| 1 | 28,75 | 3547,4 | 177,36 | |
| 2 | 36,72 | 3461,4 | 173,06 | |
| 3 | 36,07 | 3449,0 | 172,44 | |
| 4 | 22,42 | 2720,5 | 136,02 | |
| 5 | 18,28 | 2541,7 | 127,08 | |
| 6 | 69,65 | 2472,2 | 123,60 | |
| 7 | 72,70 | 2417,0 | 120,84 | |
| 8 | 79,96 | 2366,2 | 118,30 | |
| 9 | 74,07 | 2361,1 | 118,05 | |
| 10 | 69,71 | 2225,8 | 111,28 | |
| 11 | 36,71 | 2192,4 | 109,61 | |
| 12 | 66,75 | 1104,8 | 55,24 | |
| 13 | 61,21 | 1057,0 | 52,84 | |
| 22 | 71,37 | 578,6 | 28,93 | |
| 23 | 50,10 | 537,7 | 26,88 | |
| 24 | 68,69 | 501,6 | 25,07 | |
| 25 | 21,20 | 0,0 | 0,00 | TMS |

Some examples are given below of preparations of the compounds according to the invention.

Example I

Preparation of pyroglutamyltryptophan

This compound was prepared according to the following reaction scheme:

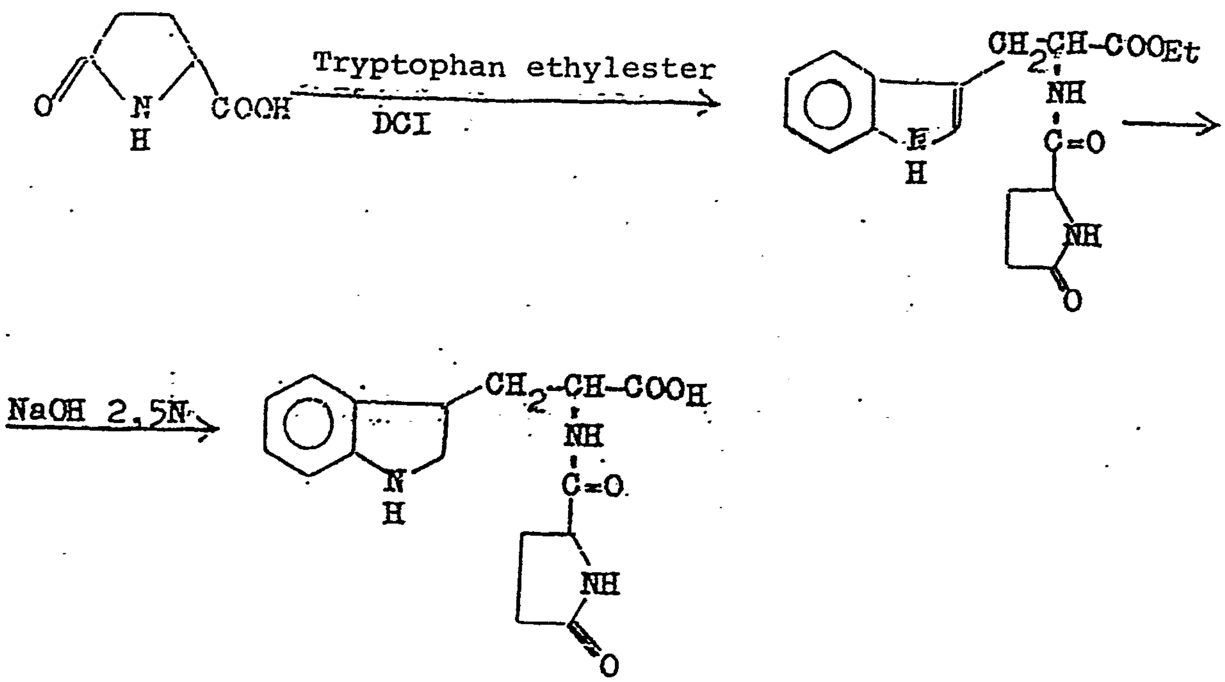


2.68g (0.01mol) of tryptophan ethyl ester hydrochloride (268.5g/mol) was dissolved in 70ml of dimethylformamide (DMF) at room temperature, and then treated in the following order with 1.01g (0.01mol) of triethylamine (101g/mol), 1.29g (1.01mol) of pyroglutamic acid (129 g/mol) and 1.06g (0.01mol) of dicyclohexylcarbodiimide (206g/mol). The reaction mixture was stirred at room temperature for 48 hours. The DMF was removed by distillation at 40°C under reduced pressure, and the residue was taken up in ethyl acetate and filtered. The solution was washed first with 40ml of 0.01N HCl, then with water until neutral. The organic phase was dried over sodium sulfate, and then evaporated to dryness; the residue so obtained was taken up in 8ml of 2.5N NaOH and stirred for 16 hours. The resulting solution was filtered through folded filter paper, extracted with

ethyl acetate and acidified with 1N HCl until pH 1.5. Upon cooling a precipitate formed which was isolated by filtration and crystallized from water.

Melting point : 130-132°C.

Elementary analysis : for $C_{16}H_{C7}N_3O_4 \cdot H_2O$.

Example II

The following example illustrates the preparation of the compounds according to the invention using the mixed anhydride method and protecting the amide nitrogen of the pyroglutamic acid with a carbobenzoxy group.

The preparation is according to the following reaction scheme:

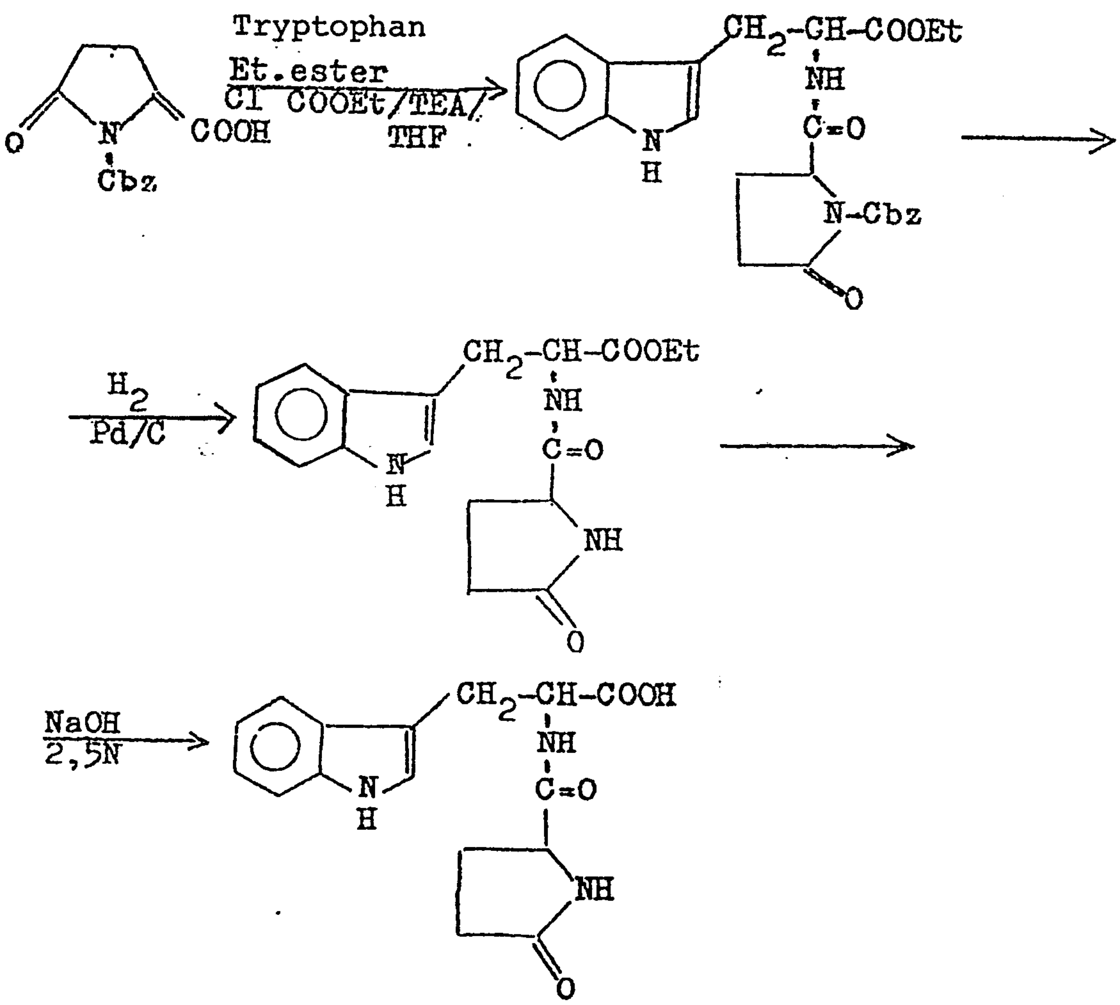

Preparation of N-carbobenzoxy-pyroglutamyltryptophan ethyl ester

1.4g (0.0053mol) of N-carbobenzoxy-pyroglutamic acid (263.3g/mol) dissolved in 50ml of anhydrous tetrahydrofuran (THF) and 0.535g (0.005mol) of triethylamine (101 g/mol) were placed at $0^oC$ in a 250ml 3 necked flask, with stirring. 0.583g (0.0053mol) of ethyl chloroformiate (ClCOOEt) were then added. The reaction mixture was held at $0^oC$ for another half hour, and then treated dropwise at the same temperature with a suspension of 1.4g (0.0052mol) of tryptophan ethyl ester hydrochloride (268.5g/mol) in 75ml of THF and 0.535ml (0.005mol) of triethylamine.

After the addition the reaction mixture was stirred at room temperature for 3 hours; the solvent was then removed at reduced pressure and the residue taken up in a mixture of water and chloroform (50/50 v/v). The organic phase was first washed with 50ml of 5% $NaHCO_3$ and then water, 0.01N HCl, and finally water until neutral. The chloroform extract was dried over sodium sulfate and evaporated under vacuum; the residue turns crystalline upon treatment with petroleum ether.
Elementary analysis : for $C_{26}H_{27}N_3O_6$.
TLC : in ethyl acetate/ethanol (2/1) Rf 0.88.

The compound is used as is, with no further character-

ization.

B) Preparation of pyroglutamyltryptophan ethyl ester

1.74g of N-carbobenzoxy-pyroglutamyltryptophan ethyl ester (477g/mol) prepared in A) was dissolved in 50ml of methanol and treated with 0.465g of palladium on carbon (10%); the resulting mixture was then treated with a stream of hydrogen for 2 hours, with stirring.

The catalyst was removed by filtration and the solution concentrated to small volume and diluted with petroleum ether (40-60°). The precipitate was collected and crystallized from a small amount of ethyl acetate. Melting point : 148-150°C.
Analysis for $C_{12}H_{21}N_3O_4$.

C) Preparation of pyroglutamyltryptophan

1.2g (0.0035mol) of pyroglutamyltryptophan ethyl ester (343g/mol) prepared in B) in 2ml of $H_2O$ and 2.8ml (0.007 mol) of 2.5N NaOH was stirred at room temperature overnight. The solution was then filtered and acidified with dilute HCl. The precipitate which separated was collected under vacuum and crystallized from water using decolorizing carbon.
Transparent needles, melting point 130-132°C
Elementary analysis for $C_{16}H_{17}N_3O_4 \cdot H_2O$.

Tests on pharmacological effects of DL-pyroglutamyl-tryptophan and ethylester thereof.

Behavioral effects of DL-pyroglutamyl-tryptophan (pGlu-Trp) have been studied in male rats of Wistar strain (purchased from Morini, Italy), weighing 130±10 g at the onset of experiments. Animals were kept under light-dark cycle (lights on between 8.00 and 20.00) with standard food and water available ad lib. Animals were injected intraperitoneally (ip) with 10 or 100 mg/kg of pGlu-Trp dissolved in a solution of $NaHCO_3$ 1 N and adjusted at pH 7.4 with HCl 0.1 N. Control animals received injection of the vehicle alone. Injections were performed 1 h prior to the behavioral testing. The animals were used only once for the behavioral experiments.

Acquisition of active avoidance behavior was studied in a single session test. The rats were trained to avoid the unconditioned stimulus (US) of a scrambled electrical footshock (0.20 mA) delivered through the grid floor. The conditioned stimulus (CS) was a buzzer presented for 5 sec prior to the US. If no escape occurred within 20 sec of CS/US presentation, the shock was terminated. A maximum of 30 conditioning trials were given with a variable intertrial interval averaging 60 sec. The learning criterion was 5 consecutive conditioned avoidance responses (CARs). For those animals that reached this criterion in less than 30 trials, the remaining trials up to a total of 30 were considered as 1ARs. Indexes of avoidance behavior were the total number of CARs and the number of learners.

Open field behavior was studied in a circular open field arena. Ambulation (number of floor units entered), rearing, grooming and defecation scores were recorded during a 5-min observation session.

Hot plate test for analgesia was studied with a circular hot plate (520°C) where the animals were placed singularly. The latency in sec to the first behavioral reaction to hot pain (licking of paw) was recorded.

Acquisition of shuttle-box active avoidance behavior appeared to be slightly decreased in rats injected ip with 10 mg/kg of pGlu-Trp as compared to controls (12.7±1.99 vs. 14.8±1.50, difference not significant). However, when pGlu-Trp was injected at the dose of 100 mg/kg, it significantly increased the number of CARs and the percentage of learners as compared to those of control rats (19.0±1.21 vs 14.2±1.47, $p<0.02$; 90.9% vs 66.6%).

Ambulation of rats injected2 ip with 10 mg/kg of pGlu-Trp appeared to be decreased as compared to that of controls (78.5±4.4 vs. 94.5±4.7). No difference was found in rearing and grooming of treated and control rats. Furthermore, defecation boli were decreased after ip injection of pGlu-Trp (10 mg/kg). Injection of pGlu-Trp (100 mg/kg) caused a change in open field behavior similar to that observed after the administration of the smaller dose.

No analgesic effect was found in rats injected ip with 10 mg/kg of pGlu-Trp and tested in hot plate test for analgesia.

Similar tests were carried out by administering analogous doses of pGlu-Trp ethyl ester, said tests showing similar results.

In conclusion it appears that an administration of pGlu-Trp or ethyl ester derivative thereof results in central effects.

The behavioral action leads to:

an improving in learning capacity;
a reduction in locomotor activity;
a reduction in defecation.

Possible clinical implications would be:

nootropic effect (improvement of learning capacity);
sedative effect;
anti-anxiety effect.

## Pharmacological action

pGlu-Trp and pGlu-Trp ethyl ester have been shown to have a pronounced neurotropic and antipsychotic effect due to the fact that the capacity of pyroglutamic acid to facilitate cross-membrane absorption of amino acids and its function as a carrier lead to higher levels of the two amino acids at the target tissues involved.

In fact, it has been shown that the peptide reaches the target with no structural changes, after which the two amino acids follow their own specific pathways and participate in the biochemical cycles involved.

This new molecule thus is of evident utility when applied in human therapy; as shown clearly in the above discussion.

## Therapeutic indications

In consideration of the pharmacological results, pGlu-Trp and pGlu-Trp ethyl ester may be found useful in human therapy, for the treatment in general of diseases of the central nervous system and in particular of depressive conditions in general, hemicrania and vasomotor headaches, pain syndromes central in origin, sleep disturbances, dementia, psychic fatigue, nocturnal enuresis, intellectual deficit, acute and chronic hepato-

pathy, chronic alcoholism, hyperammoniemia, conditions of sensory obnubilation.

Various pharmaceutical forms may be selected for these indications, containing from 500 to 1000mg of active principle, including the following preferred but in no sense limiting examples:

a) oral: capsules, tablets, pills, drinkable ampuls and effervescent packets

b) parenteral: sterile intramuscular and intravenous injectable ampuls.

Of course the pharmaceutical formulations may include in addition to the active principle, the usual pharmaceutically compatible vehicles and adjuvants, according to pharmaceutical techniques. Moreover, it is clear that the administration forms and the relative dosage schemes may be varies as required by the clinical application and by the judgment of the attending physicians.

0085283

## Claims

1. A process for the production of pyroglutamyltryptophan monohydrate comprising:

A) reacting tryptophan etyl ester hydrochloride and pyroglutamic acid in the presence of triethylamine and dicyclohexyl-carbodiimide in a dimethylformamide solution;

removing the solvent and recovering pyroglutamyl-tryptophan ethyl ester as a reaction product; and

B) reacting pyroglutamyl-tryptophan ethylester with an aqueous solution of NaOH 2.5 N;

filtering, extracting the filtrate with ethyl acetate and

adding HCl to pH 1.5;

cooling to form a precipitate;

recovering said precipitate and recrystallizing from water.

2. A process for the production of pyroglutamyl-tryptophan monohydrate, comprising:

A) reacting N-carbobenzoxy-pyroglutamic acid and tryptophan ethyl ester hydrochloride in a solution of anhydrous tetrahydrofuran, in the presence of triethylamine and ethylchloroformate at a room temperature;

removing the solvent and stirring the reaction product in a water/chloroform mixture;

recovering the organic phase so formed, drying and crystallizing N-carbobenzoxy-pyroglutamyl-tryptophan ethyl ester from petroleum ether;

reacting said compound in a methanol solution with a hydrogen stream in the presence of a palladium catalyst to form a precipitate;

recovering said precipitate and crystallizing pyroglutamyl-tryptophan ethyl ester from ethylacetate; and

B) reacting pyroglutamyl tryptophan ethyl ester with an aqueous solution of NaOH 2.5 N;

filtering, extracting the filtrate with ethyl acetate and adding HCl to pH 1.5;

cooling to form a precipitate;

recovering said precipitate and recrystallizing from water.

3. Pyroglutamyl-tryptophan monohydrate having the following characteristic bands at I.R. spectrum:

3600 $cm^{-1}$ crystallisation $H_2O$
3400 $cm^{-1}$ indole N-H
3240-3310 $cm^{-1}$ lactame and amide N-H
1730 $cm^{-1}$ free carboxy group
1630-1660 $cm^{-1}$ carbonylamide and lactame group

and showing the following characteristic bands at RMN spectrum ($^{13}C$):

things, there is a significant correlation between decreased serotonin levels and insomnia.

(c) A role for serotonin has recently been proposed in the pain function, especially with regard to headaches central in origin.

Since the presence of 5-hydroxytryptophan in the encephalon is due entirely to on-site synthesis because 5-hydroxytryptophan does not pass the hematoencephalic barrier, the importance of its precursor becomes apparent, that is tryptophan, for which there is an active transport mechanism at the cerebral level.

Therefore, the use of tryptophan was previously attempted in pathological conditions involving serotonin, such as those indicated above.

However, for example, the treatment of depressed patients with tryptophan led to contradictory results, in that the increase in plasmatic tryptophan seemed not to induce a proportional increase in the concentration of cerebral tryptophan.

It was suggested that this occurred because the tryptophan transport mechanism in the encephalon is affected by the levels of the other amino acids, which can compete with the vehicle or carrier (see A. Carlsson 'In

psycopharmacology, a generation of progress' Ed. M.A. Lipton, A. Di Mascio, K.F. Killam Raven Press, New York, pages 1057-1070, 1978).

Moreover, it has been shown that increased tryptophan concentrations lead to an activation of tryptophan pyrrolase, the first enzyme of the metabolic pathway leading to nicotinic acid, with consequent lower availability of cerebral tryptophan for serotonin synthesis (see S. Garattin, L. Valselli, 'Serotonin' Elsevier, Amsterdam, 1965).

It should also be noted that kynurenine (intermediate in the process from tryptophan to nicotinic acid) interfers with tryptophan transport in the encephalon (see T.M. Speight, G.S. Avery 'Drugs',3,159(1972) and O. Sjaastad 'Acta Neurol. Scand.',51,200(1975)). Thus increased plasma concentration of kynurenine has the effect of further lowering the level of cerebral tryptophan.

It should be kept in mind that tryptophan exerts an effect not only at the cerebral level, but also in other tissues; this can lead to an increase in the level of nicotinic acid which, as already mentioned, forms part of the pyridinic coenzymes which catalyze oxidation - reduction reactions, essential for tissue respiration.

European Patent Office

# EUROPEAN SEARCH REPORT

0085283
Application number

EP 82 83 0298

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | Chemical Abstracts, vol. 95, no. 19, 9 November 1981, Columbus, Ohio, USA N.A. FILATOVA et al. " Side reactions in the synthesis of pyroglutamylpeptides", page 803, column 2, abstract no. 169770y<br>--- | 1-8 | C 07 C 103/52<br>A 61 K 37/02 |
| X | Chemical Abstracts, vol. 92, no. 23, 9 June 1980, Columbus, Ohio, USA G.H. FISHER et al. " Structure-activity relationships for kininase II inhibition by lower homologs of the bradykinin potentiating peptide BPP9a", page 95, column 1, abstract no. 191687m & Adv. Exp. Med. Biol., 1978, pages 651-663 in combination with C.A. substance index, vol. 9 2, page 5901CS, column 2<br>--- | 1-10 | TECHNICAL FIELDS SEARCHED (Int. Cl. 3) |
| X | Chemical Abstracts, vol. 92, no. 19, 12 May 1980, Columbus, Ohio, USA L.C. MARTIN et al. " Fate of bradykinin-potentiating peptide 9a after intravenous injection", page 10, column 2, abstract no. 157471b & Biochem. J., vol. 184, no. 3, 1979, pages 713-71 6<br>--- -/- | 1-8 | C 07 C 103/52 |

The present search report has been drawn up for all claims

| Place of search | Date of completion of the search | Examiner |
|---|---|---|
| BERLIN | 16-03-1983 | BREW C.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

0085283

Application number

EP 82 83 0298



## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | Chemical Abstracts, vol. 91, no. 23, 3 December 1979, Columbus, Ohio, USA K. FUJIWARA et al. "The substrate specificity of pyrrolidone carboxylyl peptidase from Bacillus amyloliquefaciens", page 234, columns 1, 2, abstract no. 188569y & Biochim. Biophys. Acta, vol. 570, no. 1, 1979, pages 140-148<br>--- | 1-10 | |
| X | Chemical Abstracts, vol. 76, no. 17, 24 April 1972 Columbus, Ohio, USA H. SIEVERTSSON et al. "Hypothalamic hormones. 27. Synthesis of di- and tripeptides and assay in vivo for activity in the thyrotropin releasing hormone and the luteinizing releasing hormone systems", p age 64, column 1, abstract no. 94937e & J. Med. Chem., vol. 15, no. 1, 1972, pages 8-11 in combination with C.A. 9th collective Index, C hemical Substances, page 39453CS, column 2<br>----- | 1-10 | |

TECHNICAL FIELDS SEARCHED (Int. Cl. 3)

The present search report has been drawn up for all claims

| Place of search | Date of completion of the search | Examiner |
|---|---|---|
| BERLIN | 16-03-1983 | BREW C.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document